# EUROPEAN PATENT APPLICATION

(11) **EP 4 446 433 A1**
(43) Date of publication of application: **16.10.2024**
(21) Application number: 22902955.8
(22) Date of filing: 22.09.2022
(51) Int. Cl.: C12Q 1/6858, C12Q 1/6886, C12N 15/11

(54) **DIGITAL AMPLIFICATION DETECTION METHOD, DETECTION PRODUCT AND DETECTION KIT FOR SIMULTANEOUSLY IDENTIFYING MULTIPLE GENE TYPES**

(30) Priority: 09.12.2021 CN 202111503229
(71) Applicant: Nanjing Pregene Biotechnology Co., Ltd, Nanjing, Jiangsu 211899 (CN)
(72) Inventor: SHANG, Wu, Nanjing, Jiangsu 210008 (CN); WANG, Youxiang, Nanjing, Jiangsu 210008 (CN); YANG, Zhijie, Nanjing, Jiangsu 210008 (CN); ZHANG, Yiliang, Nanjing, Jiangsu 210008 (CN); JIANG, Zhengxin, Nanjing, Jiangsu 210008 (CN)
(74) Representative: Proi World Intellectual Property GmbH
(86) International application number: PCT/CN2022/120663
(87) International publication number: WO 2023/103530

(57) **Abstract**

This application discloses a digital amplification detection method for simultaneously identifying multiple genotypes, a detection product and a detection kit, and relates to the technical field of gene detection. The detection method includes: S1, extracting sample DNA; and preparing a primer-probe mixed solution, including: synthesizing primers and probes, where for each target gene, at least two probes that bind to the target gene are designed; and modifying the primers with a small molecule compound and a nucleotide sequence capable of forming a secondary structure to obtain the primer-probe mixed solution; S2, carrying out a PCR reaction; and S3, analyzing detection data to obtain genotypes at different loci.

## Description

### Technical Field

This application relates to the technical field of gene mutation detection, and more specifically, relates to a digital amplification detection method for simultaneously identifying multiple gene mutations, a detection product and a detection kit.

### Background

Multiple gene detection is a difficult problem in the industry, and most of technologies can only achieve detection of limited gene mutations at present. In recent years, some technologies can achieve detection of ten or more gene mutations, such as a multiplex ligation-dependent probe amplification (MLPA) technology, a multicolor melting curve analysis (MMCA) technology and a microfluidic technology (such as a Filmarray product), but these detection means have different technical problems. According to the MLPA technology, probe design is difficult and time-consuming, reagents are expensive, and use in combination with a capillary electrophoresis instrument and other complex instruments is required, so that application is limited. According to the microfluidic technology, use of complex instruments related to microfluidics is required, and detection sensitivity cannot meet requirements in practical application.

In a process of solving the industry problems, a digital PCR technology has been used to help improve the accuracy and signal number of gene mutation detection by some researchers. The digital PCR, as an absolute quantification technology for nucleic acid molecules, can effectively distinguish negative signals and positive signals by dividing a reaction system. Based on the technology, scientists can obtain multiple signals with different fluorescence intensities by adjusting concentrations of different gene mutation detection probes, thereby achieving detection of multiple gene mutations. However, when used for detection of the multiple gene mutations, the technology is still limited by the number of primers and probes in a same system. During detection of the multiple gene mutations, multiple groups of primers and probes need to be set. Due to increase of the number of the primers, the possibility of generating non-specific amplification products, such as primer dimers, is increased, normal binding of the probes to a sequence to be detected is affected, and the accuracy and reliability of amplification and detection results of target genes are reduced.

Therefore, a novel detection method for super-multiple gene mutations is required to be developed urgently to overcome the problems in the prior art.

### Summary

In order to further achieve accurate detection of super-multiple gene mutations, this application provides a digital amplification detection method for simultaneously identifying multiple gene mutation types, a detection product and a detection kit.

In a first aspect, this application provides a digital amplification detection method for simultaneously identifying multiple gene mutation types. The following technical schemes are adopted.

A digital amplification detection method for simultaneously identifying multiple gene mutation types includes the following steps:
S1, extracting sample DNA for later use;
and preparing a primer-probe mixed solution, including the following steps:
S11, synthesizing multiple primers and probes for amplification and detection of target gene sequences, where for each of the target gene sequence, at least two detection probes are designed and used for covering different regions of the target gene sequence, and a nucleotide sequence for synthesizing the primers includes four or less bases;
S12, adding a nucleotide sequence capable of forming a secondary structure at a 5' end of the primers, and modifying the primers with a small molecule compound to obtain primers for PCR amplification,
where the small molecule compound is selected from one or more of phosphorothioate oligonucleotide, methylated oligonucleotide, peptide nucleic acid, locked nucleic acid, hypoxanthine nucleotide or a rhodium metal inserting reagent;
S13, co-dissolving the primers for PCR amplification and the probes in a buffer solution for nucleic acid dissolution to obtain the primer-probe mixed solution;
S2, preparing a PCR reaction system and then carrying out a PCR reaction, where the PCR reaction system includes the sample DNA and the primer-probe mixed solution; and carrying out the PCR reaction; and
S3, after the PCR reaction is completed, analyzing detection data to obtain locus mutation types of different target gene sequences.

When the multiple primers and probes for PCR amplification are designed by conventional methods, non-specific amplification (cross-pairing and mismatching) usually occurs, and non-target sequence products are produced. In this application, during synthesis of the primers, four bases can be selected or only three bases are selected (missing any one of a base A, a base G, a base C or a base T) to significantly reduce the occurrence of non-specific binding during amplification. Subsequently, the nucleotide sequence capable of forming a secondary structure is added at the 5' end of the amplified primers to reduce the probability of production of dimers among the primers and reduce the occurrence of non-specific amplification. In a scheme of this application, by adopting special design of the primers in combination with addition of the nucleotide sequence capable of forming a secondary structure at the 5' end of the primers and adding the small molecule compound, the binding strength and stability of the primers and the target genes are continuously enhanced, and the occurrence of non-specific amplification is significantly reduced, so that multiple PCR can be achieved.

The small molecule compound is added to achieve the effect of improving the stability of the primers and the binding stability of the primers to the target genes, especially when the nucleotide sequence for synthesizing the primers only include three bases and one or more of the phosphorothioate oligonucleotide, the methylated oligonucleotide, the peptide nucleic acid, the locked nucleic acid, the hypoxanthine nucleotide or the rhodium metal inserting reagent are used as substitutes of the missing base. The peptide nucleic acid (PNA) can identify and bind to DNA or RNA sequences in the form of Watson-Crick base pairing to form a stable double helix structure. The locked nucleic acid (LNA) is a special double cyclic nucleotide derivative. Due to a same phosphate skeleton in structure as DNA/RNA, the LNA has a good identifying ability and strong affinity to the DNA and the RNA. The hypoxanthine nucleotide or deoxyhypoxanthine can also be used as a universal base to substitute the missing base. Therefore, when selected from one or more of the peptide nucleic acid, the locked nucleic acid, the hypoxanthine nucleotide and the deoxyhypoxanthine, the small molecule compound can be used as a universal base to substitute the missing base in synthesis of the primers so as to improve the binding stability of the primers and the target genes.

Moreover, probe design in this application is performed based on a double probe shedding technology (with reference to a method disclosed in the document "Bidshahri, Roza, et al. "Quantitative detection and resolution of BRAF V600 status in colorectal cancer using droplet digital PCR and a novel wild-type negative assay." The Journal of Molecular Diagnostics 18.2 (2016): 190-204."). In an existing single probe shedding technology, two probes need to be designed, including a shedding probe and a reference probe. The shedding probe is only complementary to a wild-type sequence and covers a possible mutation locus sequence, and the reference probe is used for hybridizing with a neighboring locus of a mutation locus and is complementary to mutant and wild-type alleles, so as to achieve identification and detection of different genotypes. However, in the single probe shedding technology, only one of the two probes is used for identifying wild-type genes, so that detection efficiency is low.

Therefore, in the probe shedding technology of this application, at least two probes are set for each of the target genes, and no reference probes are set in the set probes. Multiple different probes are bound to the target genes and cover different possible mutation regions of the target genes so as to achieve comprehensive detection of mutation loci of the target genes, so that use efficiency of the probes is improved, simultaneous detection of multiple mutation types in the target genes can be achieved by one detection, and the technology has more efficient reagent design, a simpler process and a lower cost.

Optionally, the nucleotide sequence for synthesizing the primers includes three bases and does not include a base G and a base C simultaneously.

Optionally, the small molecule compound is connected to the primers by an additional addition method; and S12 includes the following steps: adding a nucleotide sequence capable of forming a three-dimensional structure at the 5' end of the primers to obtain initially modified primers; adding the initially modified primers to a buffer solution, where the buffer solution includes a small molecule compound; and carrying out a reaction to obtain the primers for PCR amplification. The buffer solution includes a reaction solution and a stop solution. The reaction solution can be selected from, for example, [Rh(NH₃)₄(phen)][OTf]₃ (0.500 g, 0.626 mmol), chrysene-5,6-dione (0.162 g, 0.626 mmol), 1:9 H₂O:MeCN (400 mL) or 1 M solution of NaOH (1.5 mL). The stop solution can be selected from 1 M solution of HCl (1.5 mL). The "adding a nucleotide sequence capable of forming a three-dimensional structure at the 5' end of the primers" is performed by a conventional method in the art, such as a method disclosed in the document "Ronaghi, Mostafa, et al. "PCR-introduced loop structure as primer in DNA sequencing." Biotechniques 25.5 (1998): 876-884.".

Optionally, a concentration of the small molecule compound in the buffer solution is 4-12 µM, and a molar ratio of the small molecule compound to the initially modified primers is (0.7-1):1.

By adopting the above technical schemes, when the rhodium metal inserting reagent is selected as the small molecule compound, the small molecule compound is added to the buffer solution and then added to the primers. A further addition method includes: first, synthesizing primers when preparing primers for PCR amplification; then, connecting a nucleotide sequence capable of forming a three-dimensional structure at the 5' end of the primers to obtain initially modified primers; and finally, mixing the obtained initially modified primers with the buffer solution containing the small molecular compound to achieve the purpose of adding the small molecular compound to the initially modified primers so as to obtain the primers for PCR amplification at last. The small molecule compound is added for the purpose of improving the binding strength of the primers and a template binding region. In the scheme, an appropriate amount of the small molecule compound is added to achieve excellent effects of reducing the mismatching probability and improving the binding strength.

Optionally, the sample DNA includes cfDNA.

Generally, a length of the cfDNA is 150-200 bp; and efficient and accurate detection of gene mutations on short-chain nucleotide fragments can be achieved in one example scheme of this application.

Optionally, a nucleotide sequence length of the probes is 10-30 bp.

By using the above technical schemes and the probes with a shorter nucleotide sequence, the PCR amplification efficiency is higher, and the base mismatching possibility is significantly reduced. In addition, the shorter probes can make fluorescein and a quenching group close enough to reduce background fluorescence, thereby achieving more accurate detection results. Meanwhile, the shorter probes are more suitable for detecting small-fragment cfDNA in the blood.

Optionally, the PCR reaction system does not include deoxycytidine triphosphate.

Optionally, in the primer-probe mixed solution, a concentration of each of the primers for PCR amplification is 7-12 µM, and a concentration of each of the probes is 7-12 µM.

Optionally, when a reaction is carried out in step S12, heating is performed to 50-80°C first, and heat preservation is performed; and then cooling is performed to 28-32°C, and heat preservation is performed.

Optionally, multiple probes do not include reference probes.

In a second aspect, an example of this application provides a detection product for detecting multiple genotypes by using the detection method.

In a third aspect, an example of this application provides a detection kit for detecting multiple genotypes by using the detection method.

Optionally, the detection kit includes the primer-probe mixed solution; the primer-probe mixed solution includes the primers for PCR amplification and the probes; and each of the target genes correspondingly has at least two of the probes.

In summary, this application has the following beneficial effects.

This application is based on a digital PCR technology. On the basis of conventional synthetic primers, only three bases are selected first to significantly reduce non-specific binding during amplification. Moreover, the nucleotide sequence capable of forming a three-dimensional structure is added at the 5' end of the primers, and the small molecule compound is introduced, so that production of dimers among the primers can be significantly reduced, non-specific amplification is reduced, the binding specificity, binding strength and binding stability of the primers to a template can be improved, and multiple amplification of a single reaction system can be achieved. Probe design is performed based on a double probe shedding technology. At least two probes are set for each of the target genes, and no reference probes are set in the set probes. Multiple different probes are bound to the target genes and cover different regions of the target genes so as to achieve comprehensive detection of mutation loci of the target genes, so that simultaneous detection of multiple genotypes or gene mutations of the target genes is achieved by one detection, and a detection process is more efficient.

In this application, a nucleotide sequence length of the probes is selected as 10-30 bp. By using the probes with a shorter nucleotide sequence, the PCR amplification efficiency is higher, and the base mismatching possibility is significantly reduced. In addition, due to low background fluorescence during detection, detection results are more accurate. Moreover, the shorter probes are more suitable for detecting small-fragment cfDNA in the blood.

### Brief Description of Figures

FIG. 1 is a schematic diagram of a mutation detection principle according to a technical scheme of this application;
FIG. 2 is a cluster analysis diagram of mutation loci of a lung cancer gene locus KRAS detected in Example 1 of this application;
FIG. 3 is a cluster analysis diagram of mutation loci of colorectal cancer gene loci PIK3CA and BRAF detected in Example 4 of this application; and
FIG. 4 is a diagram showing detection results of a cell line positive sample in Example 6 of this application.

### Detailed Description

This application is further described in detail below in conjunction with the accompanying drawings and examples.

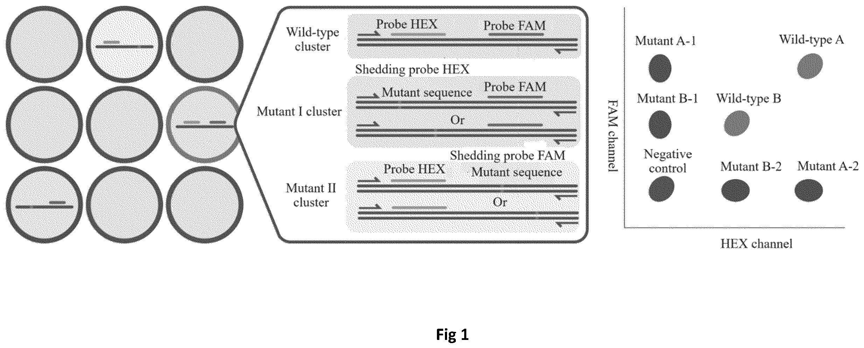

As shown in FIG. 1, at least two probes are designed in this application, and multiple different probes are bound to target genes and cover different regions of the target genes so as to achieve comprehensive detection of mutation loci of the target genes. For different mutants, clusters with different fluorescence colors appear at different positions on a final cluster analysis diagram, where the abscissa and the ordinate represent corresponding fluorescence clusters of different mutant genes appearing in different channels; and the diagonal line represents fluorescence clusters of corresponding wild-type genes.

### Example 1 Digital amplification detection method for multiple gene mutations related to lung cancer and a detection kit

A digital amplification detection method for simultaneously identifying multiple gene mutations related to lung cancer includes the following steps.

S1. Sample DNA was extracted, and a primer-probe mixed solution was prepared.

### I. Extraction of the sample DNA

Enrolled cases: Patients with pulmonary nodules treated in the Department of Thoracic Surgery of Shanghai Zhongshan Hospital from January 2020 to December 2020 were studied, where the patients were not diagnosed as having lung cancer or benign pulmonary diseases according to test results of chest CT and hematologic tumor indicators of lung cancer (including KRAS). All the patients selected to receive surgical treatment. 50 mL of peripheral venous blood was collected before surgery, and samples were divided into a lung cancer group and a benign pulmonary disease group (the benign pulmonary diseases include inflammatory pseudotumor, sclerosing hemangioma, pulmonary tuberculoma and the like) according to postoperative pathological results of tumors.

Research method: cfDNA mutation conditions of the peripheral blood of the two groups of samples were studied by using a dPCR technology, specifically including the following operation steps:
(1) 20 mL of plasma and white blood cells were collected and isolated from the peripheral venous blood;
(2) the collected samples were subjected to centrifugation at 4°C at 1,900 g for 10 min to obtain a plasma layer;
(3) centrifugation was performed at 4°C at 16,000 g for 10 min to remove cell residues; and
(4) cfDNA was obtained by using a cfDNA extraction kit (QIAamp Circulating Nucleic Acid Kit (Qiagen, Valencia, CA, USA)).

Research results: About 48.1% of the enrolled patients with lung cancer are found to have one or more mutations in a KRAS gene. In all of the patients with lung cancer detected with KRAS gene mutations, it is found by a chi-square test that the patients with benign pulmonary diseases have significant statistic differences.

KRAS mutant genes and mutation loci of the patients with lung cancer are shown in Table 1. In a reaction system, 61 mutation loci in the KRAS gene can be detected by using a digital PCR technology as well as special primers, probes and a buffer solution in this example.

**Table 1 Mutation loci of target genes related to lung cancer detected in this application**

| Genoty pe | Mutation locus | | | | | |
|---|---|---|---|---|---|---|
| KRAS | c.437C>T | c.436G>A | c.436G>C | | | |
| (A) | p.A146V | p.A146T | p.A146P | | | |
| KRAS | c.183A>C | c.183A>T | c.182A>C | c.182A>G | c.182A>T | c.181C>A |
| (B) | p.Q61H | p.Q61H | p.Q61P | p.Q61R | p.Q61L | p.Q61K |
| | c.181C>G | c.180_181TC >CA | c.180_181TC >AA | c.176C>G | c.176C>A | c.175G>A |
| | p.Q61E | p.Q61K | p.Q61K | p.A59G | p.A59E | p.A59T |
| | c.173C>T p.T58I | | | | | |
| KRAS (C-1) | c.43G>A | c.40G>A | c.39_40insG GC | c.39C>G | c.39C>T | c.39C>A |
| | p.G15S | p.V14I | p.G13_V14in sG | p.G13G | p.G13G | p.G13G |
| | c.38_39GC>TT | c.38_39GC>T G | c.38_39GC>AT | c.38_39GC> AA | c.37_39GGC> CGT | c.38G>A |
| | p.G13V | p.G13V | p.G13D | p.G13E | p.G13R | p.G13D |
| | c.38G>C | c.38G>T | c.37G>T | c.37G>A | c.37G>C | c.36_37insGGT |
| | p.G13A | p.G13V | p.G13C | p.G13S | p.G13R | p.G12_G13i nsG |
| | c.36_37TG>AT | c.36T>C | c.36T>A | c.35_36GT> AC | c.35_36GT>TC | c.35_36GT> AA |
| | p.G13C | p.G12G | p.G12G | p.G12D | p.G12V | p.G12E |
| | c.34_36GGT>T GG | c.34_36GGT> TGC | c.35G>T | c.35G>A | c.35G>C | c.34_35GG> TA |
| | p.G12W | p.G12C | p.G12V | p.G12D | p.G12A | p.G12Y |
| | c.34_35GG>AT | c.34_35GG>T T | c.34_35GG>C T | c.34G>T | c.34G>A | c.34G>C |
| | p.G12I | p.G12F | p.G12L | p.G12C | p.G12S | p.G12R |
| | c.33_34insGGA | c.32C>T | c.31G>C | c.30_31insG | c.24A>G | |
| | GCT | | | GA | | |
| | p.A11_G12insG A | p.A11V | p.A11P | p.G10_A11i nsG | p.V8V | |
| KRAS | c.64C>A | c.57G>C | c.57G>T | c.53C>A | | |
| (C-2) | p.Q22K | p.L19F | p.L19F | p.A18D | | |

Research conclusion: The KRAS gene mutations are found in the cfDNA of the peripheral blood of the patients with lung cancer, and the mutant genes include the mutation loci described in Table 1. Specific locus mutations in the KRAS gene can provide a reference for clinical diagnosis and medication guidance of the patients with lung cancer, and provide more reasonable and effective individualized guidance for the patients with lung nodules who are not diagnosed according to imaging and tumor indicators.

### II. Preparation of the primer-probe mixed solution

S11. Multiple primers and probes for amplification and detection of target genes were synthesized, where for each of the target genes, at least two detection probes were designed, and a nucleotide sequence for synthesizing the primers only includes three bases and does not include a base A and a base T simultaneously.

Specifically, the specific primers and probes were designed based on the mutation loci of KRAS according to a primer and probe design principle (such as a principle disclosed in the document "Bustin, Stephen A., Reinhold Mueller, and Tania Nolan. "Parameters for successful PCR primer design." Quantitative Real-Time PCR. Humana, New York, NY, 2020. 5-22.") and a human KRAS wild-type gene sequence published in Cosmic data, as shown in Table 2. In a mutant fluorescence probe, a 5' end was connected with a fluorescence reporter group FAM, and a 3' end was connected with a fluorescence quenching group BHQ1. In a wild-type fluorescence probe, a 5' end was connected with a fluorescence reporter group HEX, and a 3' end was connected with a fluorescence quenching group BHQ2. The fluorescence reporter groups and the quenching groups can also be reasonably selected according to a specific platform.

**Table 2 Primer-probe sequences for detection of lung cancer gene mutation loci in a reaction tube**

| Primer-probe name | Primer-probe sequence | Sequence number |
|---|---|---|
| KRAS-F1 | CCGCCGCGGCCGCCGCCT | SEQ ID NO.1 |
| KRAS-R1 | CGCCGCCGCGTCCGCGC | SEQ ID NO.2 |
| KRAS-F2 | CCCGCCCCTCCGGGG | SEQ ID NO.3 |
| KRAS-R2 | CCGGGGCGCCGCGGG | SEQ ID NO.4 |
| KRAS-P1 | TGCCGCCGCCGCTGCTGCCT | SEQ ID NO.5 |
| KRAS-P2 | CGCCACCTTCGCCG | SEQ ID NO.6 |
| KRAS-P3 | GTACCTCTCTCCC | SEQ ID NO.7 |
| KRAS-P4 | GGAAGCAGCA | SEQ ID NO.8 |

The primers and the probes for the target gene loci shown in Table 1 were designed to obtain initially modified primers for amplification and then sent to Sangon Biotech (Shanghai) Co., Ltd. for synthesis.

S12. The primers were modified for later use, and modification includes the following steps.

S121. A nucleotide sequence "CCCTGGGGGAGTATTGCGGAGGAAGGG (as shown in SEQ ID NO. 9)" was added at the 5' end of the designed primers to form a three-dimensional structure so as to obtain the initially modified primers for amplification.

S122. The synthesized initially modified primers were added to a buffer solution and heated to 70°C, heat preservation was performed for 5 min, then cooling was performed to 30°C, and heat preservation was performed for 25 min to obtain primers for PCR amplification.

The buffer solution includes the following components: 290 mM of NaC1, 4.5 mM of MgC1₂ and 18 mM of Tris (pH 7.3). The buffer solution further includes 10 µM of a small molecule compound, the small molecule compound was a rhodium metal inserting reagent, and a molar ratio of the rhodium metal inserting reagent to the initially modified primers was 0.7:1. Through analysis of X-ray single crystal diffraction data, a 5' end sequence of a detected nucleotide sequence was determined to have a three-dimensional structure. A rhodium metal used in this example has a structural formula of [RhCl₃(C10H₈N₂){(CH₃)₂SO}](CH₃)2SO, and a synthesis method refers to the document "Caruso, Francesco, et al. "Synthesis, structure, and antitumor activity of a novel tetranuclear titanium complex." Journal of medicinal chemistry 43.20 (2000): 3665-3670.".

S13. An upstream primer and a downstream primer (namely the primers for PCR amplification in S122) used for detecting the loci shown in Table 1 as well as a fluorescence probe 1 and a fluorescence probe 2 (namely the probes synthesized in S11) used for detecting the loci shown in Table 1 were co-dissolved in a TE solution to prepare the primer-probe mixed solution. Concentrations of all the upstream primer, the downstream primer, the fluorescence probe 1 and the fluorescence probe 2 in the primer-probe mixed solution were 10 µM.

A preparation method for the primer-probe mixed solution includes: diluting dry powders of four components, including the upstream primer, the downstream primer, the fluorescence probe 1 and the fluorescence probe 2, with a TE buffer solution for later use until concentrations of the probes and the primers were 100 µM, respectively.

S2. A PCR reaction system was prepared, and then a PCR reaction was carried out, where the PCR reaction system includes the sample DNA and the primer-probe mixed solution.

Specifically, the PCR reaction system was prepared according to Table 3.

**Table 3 Reaction system (20 µL of total volume)**

| Component | Final concentration | Addition amount |
|---|---|---|
| PCR Mix | / | 10µL |
| Upstream primer (10 µM) | 0.4µM | 0.8µL |
| Downstream primer (10 µM) | 0.4µM | 0.8µL |
| Fluorescence probe 1 (10 µM) | 0.2µM | 0.4µL |
| Fluorescence probe 2 (10 µM) | 0.2µM | 0.4µL |
| Template DNA | 1ng/µL | 2µL |
| ddH₂O | / | 5.6µL |

A PCR Mix was purchased from NEB and added with Triton-X-100 with a final concentration of 0.1%, 1 U of thermally stable pyrophosphatase and 5 µg/µL of BSA. According to the sequence of ddH₂O, the PCR mix, the probes, the primers and a template DNA, the above samples were added to the reaction system shown in Table 3 in a 0.2 mL PCR tube according to an addition amount of 20 µL, a mixed system was evenly mixed by gentle swirling for 15 s, and a solution was collected at the bottom of the test tube by transient centrifugation. Prepared reaction systems with different proportions were loaded to a PCR chip to form a micro-reaction unit. The chip was placed in a digital PCR instrument, and a PCR reaction was carried out according to PCR reaction conditions shown in Table 4.

The PCR Mix does not include a deoxycytidine triphosphate component.

**Table 4 PCR reaction conditions**

| Step | Tem perature | Time | Cycle number |
|---|---|---|---|
| Predenaturation | 95°C | 300 | 1 |
| Denaturation | 95°C | 15 | 40 |
| Annealing | 60°C | 15 | |
| Extension | 60°C | 30 | |

S3. After the PCR reaction was completed, detection data were analyzed to obtain genotypes at different loci.

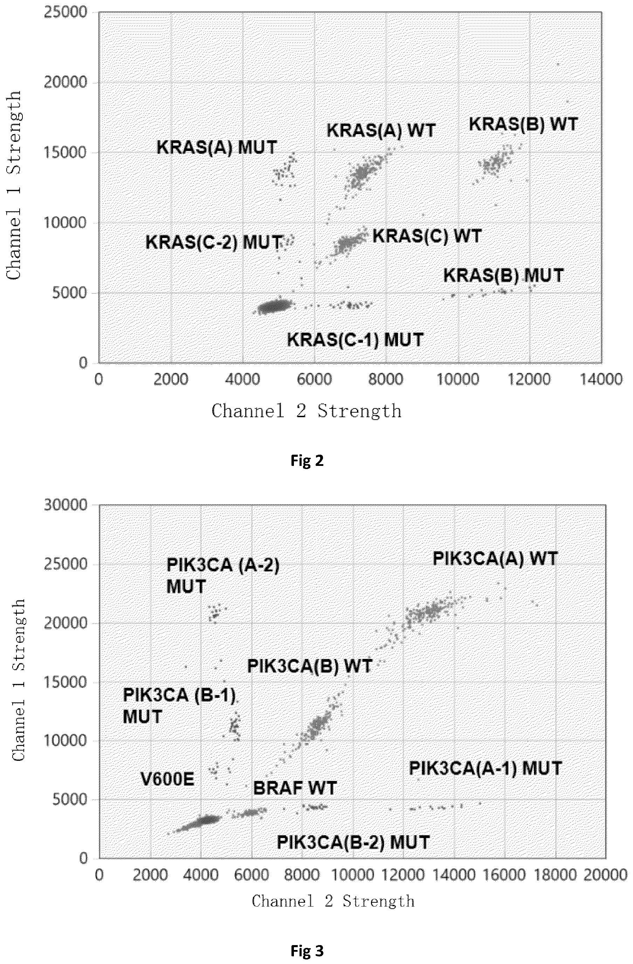

Specifically, after amplification was completed, effective positive fluorescence points of two channels were interpreted by computer analysis, and results were analyzed. As shown in FIG. 2, FIG. 2 is a diagram showing test results of clinical samples (namely cluster analysis of mutation loci of a lung cancer gene locus KRAS), the ordinate represents an FAM fluorescence channel, and the abscissa represents an HEX fluorescence channel. As can be seen from analysis of test results, corresponding signals can be detected by cluster analysis, which correspond to all of the 61 gene loci shown in Table 1.

Mutation abundance of a target gene group can be calculated by dividing a mutant signal (MUT) by a corresponding wild-type signal (WT). Further, by comparing the consistency of results of this example with results of existing methodology (NGS), it is found that a total of 143 cases of clinical blood samples were detected in this example (each case was tested by NGS), in which 133 cases have consistency with the NGS results, 10 cases have no consistency, and a consistency rate is 93%.

A detection kit for simultaneously identifying multiple gene mutations related to lung cancer includes primers and probes for PCR amplification, a TE buffer solution and a PCR mixed solution.

The PCR mixed solution was purchased from NEB and added with Triton-X-100 with a final concentration of 0.1%, 1 U of thermally stable pyrophosphatase and 5 µg/µL of BSA, and further includes a PCR mix, where the PCR mix does not include a deoxycytidine triphosphate component.

The detection kit further includes positive quality control products and negative quality control products.

A preparation method for the positive quality control products includes: selecting 200 bp of a wild-type sequence and a mutant sequence at each gene mutation locus shown in Table 1, and loading the sequences into a plasmid vector pET-23d(+)(Promega) after synthesis, respectively; performing quantification by using Qubit 3.0, calculating a copy number concentration of two types of plasmids, and mixing the two plasmids at a copy number ratio of 1:3,000, 1:2,000, 1:1,000, 1:500, 1:200, 1:100, 1:50 and 1:10; and then, breaking a plasmid mixture into fragments of about 180 bp by ultrasonic treatment, and subjecting the fragments to quantification to 20 ng/µL to serve as the gradient positive quality control products.

The negative quality control products are composed of the wild-type plasmid separately. Fragments of about 180 bp were obtained by breaking using a same method and then subjected to quantification to 20 ng/µL to serve as the negative quality control products.

### Example 2 Digital amplification detection method for multiple gene mutations related to lung cancer and a detection kit

A digital amplification detection method for simultaneously identifying multiple gene mutations related to lung cancer is provided. Different from Example 1, the selected small molecule compound was directly added to the primers in this example, and that is to say, the selected primers were obtained after modification with locked nucleic acid. In addition, in step S122, no small molecule compound was added to the buffer solution.

The locked nucleic acid was commercially available, which was a locked nucleic acid oligomer chain purchased from Beijing SBS Genetech Co., Ltd.

Other conditions were the same as those in Example 1.

A detection kit for simultaneously identifying multiple gene mutations related to lung cancer is provided.

Different from Example 1, the primers for PCR amplification were prepared in this example, and other conditions were the same as those in Example 1.

### Example 3 Digital amplification detection method for multiple gene mutations related to lung cancer and a detection kit

A digital amplification detection method for simultaneously identifying multiple gene mutations related to lung cancer is provided.

Different from Example 1, peptide nucleic acid (purchased from Beijing SBS Genetech Co., Ltd.) was selected as the small molecule compound in this example, and the peptide nucleic acid was added in step S2. Specifically, the peptide nucleic acid with a final concentration of 0.35 µM was added to the PCR Mix. In addition, in step S122, no small molecule compound was added to the buffer solution.

Other conditions were the same as those in Example 1.

A detection kit for simultaneously identifying multiple gene mutations related to lung cancer is provided.

Different from Example 1, the primers for PCR amplification were prepared in this example, and other conditions were the same as those in Example 1.

Finally, accurate detection of mutant genes (such as the 61 mutation loci in the KRAS gene shown in Table 1) related to lung cancer can also be achieved by using the method.

### Example 4 Digital amplification detection method for multiple gene mutations related to colorectal cancer and a detection kit

A digital amplification detection method for simultaneously identifying multiple gene mutations related to colorectal cancer includes the following steps.

S1. Sample DNA was extracted, and a primer-probe mixed solution was prepared.

### I. Extraction of the sample DNA

Enrolled cases: Patients with colorectal cancer treated in the Department of Anorectal Surgery of Shanghai Zhongshan Hospital from January 2019 to December 2019 were studied, where the patients were not diagnosed as having colorectal cancer or benign colorectal diseases according to test results of CT and hematologic tumor indicators of colorectal cancer (including PIK3Ca and BRAF). All the patients selected to receive surgical treatment. 50 mL of peripheral venous blood was collected before surgery, and samples were divided into a colorectal cancer group and a benign colorectal disease group according to postoperative pathological results of tumors.

Research method: cfDNA mutation conditions of the peripheral blood of the two groups of samples were studied by using a dPCR technology, specifically including the following operation steps:
(1) 20 mL of plasma and white blood cells were collected and isolated from the peripheral venous blood;
(2) the collected samples were subjected to centrifugation at 4°C at 1,900 g for 10 min to obtain a plasma layer;
(3) centrifugation was performed at 4°C at 16,000 g for 10 min to remove cell residues; and
(4) cfDNA was obtained by using a cfDNA extraction kit (QIAamp Circulating Nucleic Acid Kit (Qiagen, Valencia, CA, USA)).

Research results: In the enrolled patients with colorectal cancer, some patients were detected to have one or more mutations in PIK3CA and BRAF genes, and the patients with benign colorectal diseases were not found to have PIK3CA and BRAF gene mutations. In all of the patients with colorectal cancer detected with PIK3CA and BRAF gene mutations,

it is found by a chi-square test that the patients with benign colorectal diseases have significant statistic differences.

PIK3CA and BRAF mutant genes and mutation loci of the patients with colorectal cancer are shown in Table 5. In a reaction system, a total of 41 mutation loci of the 2 genes, namely PIK3CA and BRAF, can be detected by using a digital PCR technology as well as special primers and a buffer solution in this example.

**Table 5 Mutation loci of target genes detected in this example**

| Genotype | Mutation locus | | | | | |
|---|---|---|---|---|---|---|
| PIK3CA | c.1616C>G | c.1624G>C | c.1624G>A | c.1625A>T | | |
| (A-1) | p.P539R | p.E542Q | P.E542K | p.E542V | | |
| PIK3CA | c.1631C>A | c.1633G>C | c.1633G>A | c.1634A>C | c.1634A>G | c.1635G>C |
| (A-2) | p.T544N | p.E545Q | p.E545K | P.E545A | p.E545G | P.E545D |
| | c.1635G>T | c.1634A>T | c.1636C>A | c.1637A>G | c.1637A>T | c.1636C>G |
| | P.E545D | p.E545V | p.Q546K | p.Q546R | p.Q546L | p.Q546E |
| | c.1637A>C | c.1638G>T | c.1645C>T | | | |
| | p.Q546P | p.Q546H | p.D549N | | | |
| PIK3CA | c.3104C>T | c.3115T>C | c.3120G>A | | | |
| (B-1) | p.A1035V | p.F1039L | p.M1040I | | | |
| PIK3CA (B-2) | c.3127A>G | c.3128T>C | c.3129G>A | c.3129G>T | c.3129G>C | c.3130A>G |
| | p. M 1043V | P.M1043T | p.M1043I | p.M1043I | p.M1043I | p. N 1044 D |
| | c.3132T>A | c.3133G>A | c.3136G>A | c.3137C>A | c.3139C>T | c.3140A>G |
| | p. N 1044 K | p.D1045N | p.A1046T | p.A1046E | p. H 1047Y | p.H1047R |
| | c.3140A>T | c.3141T>G | c.3143A>G | c.3145G>C | c.3145G>A | c.3146G>C |
| | p.H1047L | p.H1047Q | p.H1048R | P.G1049R | P.G1049S | p.G1049A |
| BRAF | c.1799T>A | | | | | |
| | p.V600E | | | | | |

Research conclusion: The PIK3CA and BRAF gene mutations are found in the cfDNA of the peripheral blood of the patients with colorectal cancer, the mutant genes include the mutation loci described in Table 5, and mutations are not found in benign tumors. Specific locus mutations in the PIK3CA and BRAF genes can provide a reference for diagnosis and differential diagnosis of the colorectal cancer, and provide more reasonable and effective individualized guidance for the patients with colorectal cancer who are not diagnosed according to imaging and tumor indicators.

### II. Preparation of the primer-probe mixed solution

S11. Multiple primers and probes for amplification and detection of target genes were synthesized, where for each of the target genes, at least two detection probes were designed, and a nucleotide sequence for synthesizing the primers only includes three bases and does not include a base G and a base C simultaneously.

Specifically, design of the primers and the probes includes the following steps.
1. First, a cDNA sequence containing the PIK3CA and BRAF gene mutation loci was determined in an Ensemble database.
2. An exon sequence corresponding to the cDNA sequence on DNA was determined by BLAST.
3. According to the obtained exon sequence, a corresponding complete DNA sequence (including an exon and an intron) was determined by GenomeBrower, where the PIK3CA and BRAF mutation loci and mutant genes are shown in Table 6.
4. According to a primer and probe design principle, the primers and the probes that cover the target gene loci shown in Table 5 were designed by using an ION AMPLISEQ DESIGNER on-line design website with the DNA sequence corresponding to the PIK3CA and BRAF mutant genes as a template, as shown in Table 6. In a mutant fluorescence probe, a 5' end was connected with a fluorescence reporter group FAM, and a 3' end was connected with a fluorescence quenching group BHQ1. In a wild-type fluorescence probe, a 5' end was connected with a fluorescence reporter group CY3, and a 3' end was connected with a fluorescence quenching group BHQ2. The fluorescence reporter groups and the quenching groups can also be reasonably selected according to a specific platform.

**Table 6 Primer-probe sequences for detection of colorectal cancer gene mutation loci in a reaction tube**

| Primer-probe name | Primer-probe sequence | Sequence number |
|---|---|---|
| PIK3CA-F1 | ACATAAAAAAATA | SEQ ID NO.10 |
| PIK3CA-R1 | AAAAATTCAATTTCTAA | SEQ ID NO.11 |
| PIK3CA-F2 | AAATATTTTATAAT | SEQ ID NO.12 |
| PIK3CA-R2 | AACTTCAAAACAAACAAAACAAAACACA | SEQ ID NO.13 |
| PIK3CA-P1 | TGAGATAACCTG | SEQ ID NO.14 |
| PIK3CA-P2 | GCAGTAGAAATAATC | SEQ ID NO.15 |
| PIK3CA-P3 | GATAGTAATACCACTCTGTC | SEQ ID NO.16 |
| PIK3CA-P4 | TTCAGATTAGAATACCATTTCTTA | SEQ ID NO.17 |
| BRAF-F1 | CACATAAACAAATTTT | SEQ ID NO.18 |
| BRAF-R1 | ATAATTAAAAATTTCCA | SEQ ID NO.19 |
| BRAF-P1 | ACACGGTATT | SEQ ID NO.20 |

The primers and the probes for the target gene loci shown in Table 6 were sent to Sangon Biotech (Shanghai) Co., Ltd. for synthesis.

S12. The primers were modified for later use, and modification includes the following steps.

S121. A nucleotide sequence "TCCTCATCTGTCGAGACTCACTATAGGAAGAGATGTCAACTCGTGCACGAGTT GACATCTCTTCTCCGAGCCGGTCGAAATATTGGAGGAAGCTCGAGCTGGAGGA AAAGTGAGTCTCGACAGATGAGGA (as shown in SEQ ID NO. 21)" was added at the 5' end of the designed primers to form a three-dimensional structure at a front end of the primers so as to obtain initially modified primers for amplification.

S122. The synthesized initially modified primers were added to a buffer solution and heated to 70°C, heat preservation was performed for 5 min, then cooling was performed to 30°C, and heat preservation was performed for 25 min to obtain primers for PCR amplification.

The buffer solution includes the following components: 300 mM of NaC1, 5 mM of MgC1₂ and 20 mM of Tris (pH 7.6). The buffer solution further includes 5 µM of a small molecule compound, the small molecule compound was methylated oligonucleotide (purchased from Genscript Biotech Co., Ltd.), and a molar ratio of the methylated oligonucleotide to the initially modified primers was 0.8:1. Through analysis of X-ray single crystal diffraction data, a 5' end sequence of a detected nucleotide sequence was determined to have a three-dimensional structure.

S13. An upstream primer and a downstream primer (namely the primers for PCR amplification in S122) used for detecting the loci shown in Table 5 as well as a mutant fluorescence probe and a wild-type fluorescence probe (namely the probes synthesized in S11) used for detecting the loci shown in Table 5 were co-dissolved in a TE solution to prepare the primer-probe mixed solution. Concentrations of all the upstream primer, the downstream primer and the fluorescence probes in the primer-probe mixed solution were 10 µM.

A preparation method for the primer-probe mixed solution includes: diluting dry powders of four components, including the upstream primer, the downstream primer, the fluorescence probe 1 and the fluorescence probe 2, with a TE buffer solution for later use until concentrations of the probes and the primers were 100 µM, respectively.

S2. A PCR reaction system was prepared, and then a PCR reaction was carried out, where the PCR reaction system includes the sample DNA and the primer-probe mixed solution.

Specifically, the PCR reaction system was prepared according to Table 7.

**Table 7 Reaction system (20 µL of total volume)**

| Component | Final concentration | Addition amount |
|---|---|---|
| PCR Mix | / | 10µL |
| Upstream primer (10 µM) | 0.4µM | 0.8µL |
| Downstream primer (10 µM) | 0.4µM | 0.8µL |
| Fluorescence probe **1** (10 µM) | 0.2µM | 0.4µL |
| Fluorescence probe **2** (10 µM) | 0.2µM | 0.4µL |
| Template DNA | 1ng/µL | 2µL |
| ddH₂O | / | 5.6µL |

A PCR Mix was purchased from NEB and added with Triton-X-100 with a final concentration of 0.1%, 1 U of thermally stable pyrophosphatase and 5 µg/µL of BSA. According to the sequence of ddH₂O, the PCR mix, the probes, the primers and a template DNA, the above samples were added to the reaction system shown in Table 7 in a 0.2 mL PCR tube according to an addition amount of 20 µL, a mixed system was evenly mixed by gentle swirling for 15 s, and a solution was collected at the bottom of the test tube by transient centrifugation. Prepared reaction systems with different proportions were loaded to a PCR chip to form a micro-reaction unit. The chip was placed in a digital PCR instrument, and a PCR reaction was carried out according to PCR reaction conditions shown in Table 8.

The PCR Mix does not include a deoxycytidine triphosphate (dCTP) component.

**Table 8 PCR reaction conditions**

| Step | Tem perature | Time | Cycle number |
|---|---|---|---|
| Predenaturation | 95°C | 300 | 1 |
| Denaturation | 95°C | 15 | 40 |
| Annealing | 60 | 15 | |
| Extension | 60 | 30 | |

S3. After the PCR reaction was completed, detection data were analyzed to obtain genotypes at different loci.

Specifically, after amplification was completed, effective positive fluorescence points of two channels were interpreted by computer analysis, and results were analyzed. As shown in FIG. 3, FIG. 3 is a diagram showing test results of clinical samples (namely cluster analysis of mutation loci of colorectal cancer gene loci PIK3CA and BRAF), the ordinate represents an FAM fluorescence channel, and the abscissa represents an HEX fluorescence channel. As can be seen from analysis of test results, corresponding signals can be detected by cluster analysis, which correspond to all of the 41 gene loci shown in Table 5.

Mutation abundance of a target gene group can be calculated by dividing a mutant signal (MUT) by a corresponding wild-type signal (WT). By using the method, 108 cases of clinical blood samples were detected (each case was detected by NGS), in which 107 cases have consistency with NGS results, 2 cases have no consistency (due to different panel designs), and a consistency rate is 98%.

DNA from serum, plasma, peripheral blood, pleural effusion, body fluid or tissues was also detected by the applicant at the gene mutation loci shown in Table 5, and good repeatability is achieved. The detection method in this example can achieve sensitive detection of the colorectal cancer gene mutation loci, has no non-specific amplification and has good specificity. Multiple primers can be added for detection, so that a substrate in a reaction process can be effectively reduced.

A detection kit for simultaneously identifying multiple gene mutations related to colorectal cancer includes a primer-probe mixed solution and a PCR mixed solution.

The primer-probe mixed solution includes a buffer solution, probes in the buffer solution and initially modified primers in the buffer solution. The buffer solution includes the following components: 300 mM of NaC1, 5 mM of MgC1₂ and 20 mM of Tris (pH 7.6), and the buffer solution further includes 5 µM of a small molecule compound. The small molecule compound was locked nucleic acid, a molar ratio of the locked nucleic acid to the initially modified primers was 0.8:1, and contents of the initially modified primers and the probes in the buffer solution were 10 µM.

The PCR mixed solution was purchased from NEB and added with Triton-X-1 00 with a final concentration of 0.1%, 1 U of thermally stable pyrophosphatase and 5 µg/µL of BSA, and further includes a PCR mix, where the PCR mix does not include a deoxycytidine triphosphate component.

The kit further includes positive quality control products and negative quality control products.

A preparation method for the positive quality control products includes: selecting 200 bp of a wild-type sequence and a mutant sequence at each gene mutation locus shown in Table 5, and loading the sequences into a plasmid vector pET-23d(+)(Promega) after synthesis, respectively; performing quantification by using Qubit 3.0, calculating a copy number concentration of two types of plasmids, and mixing the two plasmids at a copy number ratio of 1:3,000, 1:2,000, 1:1,000, 1:500, 1:200, 1:100, 1:50 and 1:10; and then, breaking a plasmid mixture into fragments of about 180 bp by ultrasonic treatment, and subjecting the fragments to quantification to 20 ng/µL to serve as the gradient positive quality control products.

The negative quality control products are composed of the wild-type plasmid separately. Fragments of about 180 bp were obtained by breaking using a same method and then subjected to quantification to 20 ng/µL to serve as the negative quality control products.

### Example 5 Digital amplification detection method for multiple gene mutations related to colorectal cancer and a detection kit

Different from Example 4, in step S121 in this example, a nucleotide sequence capable of forming a three-dimensional structure was added at the 5' end of the designed primers. Different from Example 4, a nucleotide sequence "ACTCATCTGTGAGACTCACTATAGGAAGAGATGTCAACTCGTGCACGAGTTGA CATCTCTTCTCCGAGCCGGTCGAAATATTGGAGGAAGCTCGAGCTGGAGGAAA AGTGAGTCTCACAGATGAGT (as shown in SEQ ID NO. 22)" was added in this example to obtain initially modified primers for amplification.

Other steps were the same as those in Example 4. After PCR amplification, it is found that when the nucleotide sequence capable of forming a three-dimensional structure is added to the 5' end of the designed primers, non-specific amplification obviously occurs when the cycle number is 25; and when PCR amplification is performed by the scheme in Example 4, non-specific amplification still does not occur when the cycle number is at least 120.

### Example 6 Digital amplification detection method for multiple gene mutations related to esophageal squamous cell carcinoma and a detection kit

A digital amplification detection method for simultaneously identifying multiple gene mutations related to esophageal squamous cell carcinoma includes the following steps.

S1. Sample DNA was extracted, and a primer-probe mixed solution was prepared.

The sample DNA was extracted. A purchased esophageal squamous cell carcinoma cell line KYSE270 was used as negative sample, a purchased mutation cell line TP53 was used as a positive sample, and Genomic DNA was obtained by a genomic DNA extraction Kit (QIAamp DNA Mini Kit (Qiagen, Valencia, CA, USA)).

Preparation of the primer-probe mixed solution
S11. Multiple primers and probes for amplification and detection of target genes were synthesized, where for each of the target genes, at least two detection probes were designed, and a nucleotide sequence for synthesizing the primers only includes three bases and does not include a base G and a base C simultaneously.

Specifically, design of the primers and the probes includes the following steps.
1. First, a DNA sequence containing TP53 gene mutation loci was determined in an Ensemble database.
2. An exon sequence corresponding to DNA sequence on DNA was determined by BLAST.
3. According to the obtained exon sequence, a corresponding complete DNA sequence (including an exon and an intron) was determined by GenomeBrower, where the TP53 mutation loci and mutant genes are shown in Table 9.

**Table 9 Mutation loci of target genes detected in this example**

| Genotyp e | Mutation locus | | | | | |
|---|---|---|---|---|---|---|
| TP53 (A-1) | p.C277G | p.C277Y | p.C277F | p.C277* | p.C277C | p.C277 W |
| | c.829T>G | c.830G>A | c.830G>T | c.831T>A | c.831T>C | c.831T> G |
| | p.P278F | p.P278T | p.P278A | p.P278S | p.P278fs*67 | p.P278H |
| | c.832_833CC >TT | c.832C>A | c.832C>G | c.832C>T | c.832delC | c.833C> A |
| | p.P278R | p.P278L | p.P278fs* 67 | p.G279fs*65 | p.R280fs*65 | p.G279R |
| | c.833C>G | c.833C>T | c.833delC | c.835_838delG GGA | c.835deIG | c.835G> A |
| | p.G279W | p.G279E | p.G279V | p.G279G | p.R280_D281delR D | p.R280G |
| | c.835G>T | c.836G>A | c.836G>T | c.837G>A | c.838_843delAGA GAC | c.838A> G |
| | p.R280* | p.R280K | p.R280T | p.R280I | p.R280S | p.R280R |
| | c.838A>T | c.839G>A | c.839G>C | c.839G>T | c.840A>C | c.840A> G |
| | p.R280S | p.D281N | p.D281H | p.D281Y | p.D281A | G |
| | c.840A>T | c.841G>A | c.841G>C | c.841G>T | c.842A>C | c.842A> G |
| | p.D281V | p.R282W | p.D281E | p.D281E | p.D281D | p.R282R |
| | c.842A>T | c.843_844 | c.843C>A | c.843C>G | c.843C>T | c.844C> |
| | | CC>TT | | | | A |
| | p.R282G | p.R282W | p.R282Q | p.R282P | p.R282L | p.R282R |
| | c.844C>G | c.844C>T | c.845G>A | c.845G>C | c.845G>T | c.846G> A |
| TP53 (A-2) | p.N268N | p.S269G | p.S269C | p.S269_F270>I | p.S269N | p.S269T |
| | c.804C>T | c.805A>G | c.805A>T | c.806_808delG CT | c.806G>A | c.806G> C |
| | p.S269S | p.F270I | p.F270L | p.F270V | p.F270Y | p.F270S |
| | c.807C>T | c.808T>A | c.808T>C | c.808T>G | c.809T>A | c.809T> C |
| | p.F270C | p.F270L | p.F270L | p.E271K | p.E271Q | p.E271* |
| | c.809T>G | c.810T>A | c.810T>G | c.811G>A | c.811G>C | c.811G> T |
| | p.E271G | p.E271V | p.E271E | p.E271D | p.V272fs*73 | p.V272 M |
| | c.812A>G | c.812A>T | c.813G>A | c.813G>C | c.814deIG | c.814G> A |
| | p.V272L | p.V272L | p.V272E | p.V272A | p.V272G | p.R273S |
| | c.814G>C | c.814G>T | c.815T>A | c.815T>C | c.815T>G | c.817C> A |
| | p.R273G | p.R273C | p.R273fs* 72 | p.R273H | p.R273P | p.R273L |
| | c.817C>G | c.817C>T | c.817delC | c.818G>A | c.818G>C | c.818G> T |
| | p.V274I | p.V274L | p.V274F | | | |
| | c.820G>A | c.820G>C | c.820G>T | | | |
| TP53 (B-1) | p.G244G | p.G244G | p.G244G | p.G245N | p.G245fs*2 | p.G245S |
| | c.732C>A | c.732C>G | c.732C>T | c.733_734GG> AA | c.733deIG | c.733G> A |
| | p.G245R | p.G245C | p.G245D | p.G245A | p.G245V | p.G245 G |
| | c.733G>C | c.733G>T | c.734G>A | c.734G>C | c.734G>T | c.735C> T |
| | p. M 246V | p.M246L | p.M246fs* 1 | p.M246K | p.M246T | p.M246 R |
| | c.736A>G | c.736A>T | c.736delA | c.737T>A | c.737T>C | c.737T> G |
| | p.M246I | p.M246I | p.M246I | p.N247D | p.N247Y | p.N247T |
| | c.738G>A | c.738G>C | c.738G>T | c.739A>G | c.739A>T | c.740A> C |
| | p.N247S | p.N247I | p.N247_R 248>KW | p.R248W | p.N247K | p.N247 N |
| | c.740A>G | c.740A>T | c.741_742 CC>AT | c.741_742CC>T | c.741C>A | c.741C> T |
| | p.R248R | p.R248G | p.R248W | p.R248fs*97 | p.R248Q | p.R248L |
| | c.742C>A | c.742C>G | c.742C>T | c.742delC | c.743_744GG>AA | c.743_7 44GG>T |
| | p.R248Q | p.R248P | p.R248L | p.R248R | p.R248R | p.R249G |
| | c.743G>A | c.743G>C | c.743G>T | c.744G>A | c.744G>C | c.745A> G |
| | p.R249W | p.R249fs* | p.R249K | p.R249T | p.R249M | p.R249R |
| | | 96 | | | | |
| | c.745A>T | c.746delG | c.746G>A | c.746G>C | c.746G>T | c.747G> A |
| | p.R249S | p.P250A | | | | |
| | c.747G>T | c.748C>G | | | | |
| TP53 (B-2) | p.M237V | p.M237L | p.M237fs* 10 | p.M237fs*10 | p.M237K | p.M237 T |
| | c.709A>G | c.709A>T | c.709delA | c.710delT | c.710T>A | c.710T> C |
| | p.M237R | p.M237I | p.M237I | p.M237I | p.C238S | p.C238R |
| | c.710T>G | c.711G>A | c.711G>C | c.711G>T | c.712T>A | c.712T> C |
| | p.C238G | p.C238Y | p.C238S | p.C238F | p.N239fs*1 | p.C238* |
| | c.712T>G | c.713G>A | c.713G>C | c.713G>T | c.714_715insT | c.714T> A |
| | p.C238W | p.N239_S 240delNS | p.N239_C 242deINSS C | p.N239D | p.N239Y | p.N239f s*8 |
| | c.714T>G | c.715_720 delAACAG T | c.715_726 del12 | c.715A>G | c.715A>T | c. 715del A |
| | p.N239T | p.N239S | p.N239K | p.N239K | p.S240G | p.S240C |
| | c.716A>C | c.716A>G | c.717C>A | c.717C>G | c.718A>G | c.718A> T |
| | p.S240fs*7 | p.S240T | p.S240I | p.S240R | p.S240S | p.S240R |
| | c.718delA | c.719G>C | c.719G>T | c.720T>A | c.720T>C | c.720T> |
| | | | | | | G |
| | p.S241del | p.S241fs* 6 | p.S241T | p.S241P | p.S241A | p.S241F |
| | c.721_723del TCC | c.721delT | c.721T>A | c.721T>C | c.721T>G | c.722_7 23CC>T T |
| | p.S241del | p.S241Y | p.S241C | p.S241F | p.C242fs*5 | p.C242f s*5 |
| | c.722_724del CCT | c.722C>A | c.722C>G | c.722C>T | c. 722delC | c.723del C |
| | p.C242S | p.C242R | p.C242G | p.C242Y | p.C242S | p.C242F |
| | c.724T>A | c.724T>C | c.724T>G | c.725G>A | c.725G>C | c.725G> T |
| | p.C242* | p.C242W | p.C242C | p.M243L | p.M243V | p.M243 L |
| | c.726C>A | c.726C>G | c.726C>T | c.727A>C | c.727A>G | c.727A> T |
| | p.M243K | p.M243T | p.M243R | p.M243I | p.M243I | p.G244f s*3 |
| | c.728T>A | c.728T>C | c.728T>G | c.729G>A | c.729G>C | c. 730del G |
| | p.G244S | p.G244R | p.G244C | p.G244D | p.G244A | p.G244V |
| | c.730G>A | c.730G>C | c.730G>T | c.731G>A | c.731G>C | c.731G> T |
| TP53 (C-1) | p.R174fs*1 | p.R175_E 180delRC | p.C176_R1 81delCPH | p.P177fs*3 | p.P177_C182delP HHERC | p.P177P |
| | | PHHE | HER | | | |
| | c.520_536del 17 | c.523_540 del18 | c.526_543 del18 | c.529_545del1 7 | c.529_546del18 | c.531C> G |
| | P.P177P | p.H178N | p.H178D | P.H178Y | p.H178fs*69 | p.H178P |
| | c.531C>T | c.532C>A | c.532C>G | c.532C>T | c.532delC | c.533A> C |
| | P.H179Y | p.H178Q | p.H178Q | p.H178H | p.H179N | p.H179 D |
| | c.534_535CC >TT | c.534C>A | c.534C>G | c.534C>T | c.535C>A | c.535C> G |
| | P.H179Y | p.H179P | p.H179R | p.H179L | p.H179Q | p.H179 H |
| | c.535C>T | c.536A>C | c.536A>G | c.536A>T | c.537T>A | c.537T> C |
| | p.H179Q | p.E180K | p.E180* | p.E180D | p.R181C | p.R181H |
| | c.537T>G | c.538G>A | c.538G>T | c.540G>T | c.541C>T | c.542G> A |
| | p.R181P | p.R181L | p.R181R | p.C182S | p.C182R | p.C182Y |
| | c.542G>C | c.542G>T | c.543C>T | c.544T>A | c.544T>C | c.545G> A |
| | p.C182* | p.C182C | p.S183P | p.S183* | p.S183* | p.S183L |
| | c.546C>A | c.546C>T | c.547T>C | c.548C>A | c.548C>G | c.548C> T |
| TP53 (C-2) | p.V172fs*2 | p.V172I | p.V172F | p.V172D | p.V172A | p.V172G |
| | c.514delG | c.514G>A | c.514G>T | c.515T>A | c.515T>C | c.515T> G |
| | p.V172V | p.V173fs* 1 | p.V173M | p.V173L | p.V173L | p.V173A |
| | c.516T>C | c.517delG | c.517G>A | c.517G>C | c.517G>T | c.518T> C |
| | p.V173G | p.V173V | p. R174W | p.R174K | p.R174M | p.R174f s*73 |
| | c.518T>G | c.519G>A | c.520A>T | c.521G>A | c.521G>T | c.522del G |
| | p.R174R | p.R175S | p.R175G | p.R175C | p.R175H | p.R175P |
| | c.522G>A | c.523C>A | c.523C>G | c.523C>T | c.524G>A | c.524G> C |
| | p.R175L | p.R175R | p.R175R | p.C176fs*71 | p.C176S | p.C176R |
| | c.524G>T | c.525C>G | c.525C>T | c.526delT | c.526T>A | c.526T> C |
| | p.C176G | p.C176Y | p.C176S | p.C176F | p.C176* | p.C176 W |
| | c.526T>G | c.527G>A | c.527G>C | c.527G>T | c.528C>A | c.528C> G |
| | p.H178fs*69 | P.P177S | p.H178fs* 69 | p.P177H | p.P177R | p.P177L |
| | c.528delC | c.529C>T | c.529delC | c.530C>A | c.530C>G | c.530C> T |

4. According to a primer and probe design principle, the primers and the probes that cover the target gene loci shown in Table 9 were designed by using a Primer3web (version 4.1.0) on-line design website with the DNA sequence corresponding to the TP53 mutant genes as a template, as shown in Table 10. In a mutant fluorescence probe, a 5' end was connected with a fluorescence reporter group FAM, and a 3' end was connected with a fluorescence quenching group BHQ1. In a wild-type fluorescence probe, a 5' end was connected with a fluorescence reporter group CY3, and a 3' end was connected with a fluorescence quenching group BHQ2. The fluorescence reporter groups and the quenching groups can also be reasonably selected according to a specific platform.

**Table 10 Primer-probe sequences for detection of colorectal cancer gene mutation loci in a reaction tube**

| Primer-probe name | Primer-probe sequence | Sequence number |
|---|---|---|
| TP53-F1 | TGGGTGACAGAGTGAGACTTC | SEQ ID NO.23 |
| TP53-R1 | ACCCGGCCCTCATTATTTCT | SEQ ID NO.24 |
| TP53-F2 | CCAACATGGTGAAACCCCAA | SEQ ID NO.25 |
| TP53-R2 | CCGAAGTGCTGGGATTACA | SEQ ID NO.26 |
| TP53-F3 | GGCACCTCTAATCCCAGCTA | SEQ ID NO.27 |
| TP53-R3 | CCTCAGCCTCCCGAAGTG | SEQ ID NO.28 |
| TP53-P1 | ACGTTGATACCATACTGGAGGT | SEQ ID NO.29 |
| TP53-P2 | AGGATTTGACTCTCA | SEQ ID NO.30 |
| TP53-P3 | TACTCAGGAGGCTGAGGCAGGAGAA | SEQ ID NO.31 |
| TP53-P4 | TTGCTTGGACCTGGGAGGCAGAGGT | SEQ ID NO.32 |
| TP53-P5 | GGAGGCAGAGGTTGCAGTGAG | SEQ ID NO.33 |
| TP53-P6 | GAGATCGCGCTATTGCACTC | SEQ ID NO.34 |

The primers and the probes for the target gene loci shown in Table 10 were sent to Sangon Biotech (Shanghai) Co., Ltd. for synthesis.

S12. The primers were modified for later use, and modification includes the following steps.

S121. A nucleotide sequence "AATGCCAACAATGACCGCAGGTACCCAGATTCAAGGAGAACAGAAGCAGTCAG TCATGGCCCCCTGGTGTTTTTGAATAATTTGGGTAAAAAGAAGGGACGGACTGA AGATGGAGGTACGAGATTGTTGGCAC (as shown in SEQ ID NO. 35)" was added at the 5' end of the designed primers to form a three-dimensional structure at a front end of the primers so as to obtain initially modified primers for amplification.

S122. The synthesized initially modified primers were added to a buffer solution and heated to 70°C, heat preservation was performed for 5 min, then cooling was performed to 30°C, and heat preservation was performed for 25 min to obtain primers for PCR amplification.

The buffer solution includes the following components: 300 mM of NaC1, 5 mM of MgC1₂ and 20 mM of Tris (pH 7.6), and the buffer solution further includes 5 µM of a small molecule compound. The small molecule compound was phosphorothioate oligonucleotide (purchased from Sigma-Aldrich (Shanghai) Trading Co., Ltd.), and a molar ratio of the phosphorothioate oligonucleotide to the initially modified primers was 0.6:1. Through analysis of X-ray single crystal diffraction data, a 5' end sequence of a detected nucleotide sequence was determined to have a three-dimensional structure.

S13. An upstream primer and a downstream primer (namely the primers for PCR amplification in S122) used for detecting the loci shown in Table 9 as well as a mutant fluorescence probe and a wild-type fluorescence probe (namely the probes synthesized in S11) used for detecting the loci shown in Table 9 were co-dissolved in a TE solution to prepare the primer-probe mixed solution. Concentrations of all the upstream primer, the downstream primer and the fluorescence probes in the primer-probe mixed solution were 10 µM.

A preparation method for the primer-probe mixed solution includes: diluting dry powders of four components, including the upstream primer, the downstream primer, the fluorescence probe 1 and the fluorescence probe 2, with a TE buffer solution for later use until concentrations of the probes and the primers were 100 µM, respectively.

S2. A PCR reaction system was prepared, and then a PCR reaction was carried out, where the PCR reaction system includes the sample DNA and the primer-probe mixed solution.

Specifically, the PCR reaction system was prepared according to Table 11.

**Table 11 Reaction system (20 µL of total volume)**

| Component | Final concentration | Addition amount |
|---|---|---|
| PCR Mix | / | 10µL |
| Upstream primer (10 µM) | 0.4µM | 0.8µL |
| Downstream primer (10 µM) | 0.4µM | 0.8µL |
| Fluorescence probe 1 (10 µM) | 0.2µM | 0.4µL |
| Fluorescence probe 2 (10 µM) | 0.2µM | 0.4µL |
| Template DNA | 1ng/µL | 2µL |
| ddH₂O | / | 5.6µL |

A PCR Mix was purchased from NEB and added with Triton-X-100 with a final concentration of 0.1%, 1 U of thermally stable pyrophosphatase and 5 µg/µL of BSA. According to the sequence of ddH₂O, the PCR mix, the probes, the primers and a template DNA, the above samples were added to the reaction system shown in Table 11 in a 0.2 mL PCR tube according to an addition amount of 20 µL, a mixed system was evenly mixed by gentle swirling for 15 s, and a solution was collected at the bottom of the test tube by transient centrifugation. Prepared reaction systems with different proportions were loaded to a PCR chip to form a micro-reaction unit. The chip was placed in a digital PCR instrument, and a PCR reaction was carried out according to PCR reaction conditions shown in Table 12.

The PCR Mix does not include a deoxycytidine triphosphate (dCTP) component.

**Table 12 PCR reaction conditions**

| Step | Tem perature | Time | Cycle number |
|---|---|---|---|
| Predenaturation | 95°C | 300 | 1 |
| Denaturation | 95°C | 15 | 40 |
| Annealing | 60 | 15 | |
| Extension | 60 | 30 | |

S3. After the PCR reaction was completed, detection data were analyzed to obtain genotypes at different loci.

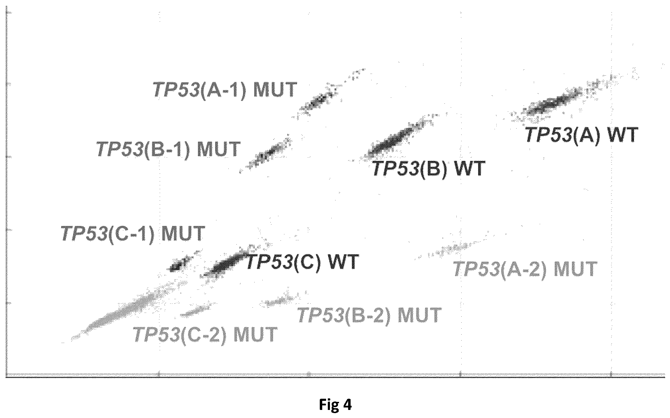

Specifically, after amplification was completed, effective positive fluorescence points of two channels were interpreted by computer analysis, and results were analyzed. As shown in FIG. 4, FIG. 4 is a diagram showing test results of the cell line positive sample (namely cluster analysis of mutation loci of an esophageal squamous cell carcinoma gene locus TP53), the ordinate represents an FAM fluorescence channel, and the abscissa represents an HEX fluorescence channel. As can be seen from analysis of test results, corresponding signals can be detected by cluster analysis, which correspond to all of the 293 gene loci shown in Table 9. Mutation abundance of a target gene group can be calculated by dividing a mutant signal (MUT) by a corresponding wild-type signal (WT).

DNA from serum, plasma, peripheral blood, pleural effusion, body fluid or tissues was also detected by the applicant at the gene mutation loci shown in Table 9, and good repeatability is achieved. The detection method in this example can achieve sensitive detection of the esophageal squamous cell carcinoma gene mutation loci, has no non-specific amplification and has good specificity. Multiple primers can be added for detection, so that a substrate in a reaction process can be effectively reduced.

A detection kit for simultaneously identifying multiple gene mutations related to esophageal squamous cell carcinoma includes a primer-probe mixed solution and a PCR mixed solution.

The primer-probe mixed solution includes a buffer solution, probes in the buffer solution and initially modified primers in the buffer solution. The buffer solution includes the following components: 300 mM of NaC1, 5 mM of MgCl₂ and 20 mM of Tris (pH 7.6). The buffer solution further includes 5 µM of a small molecule compound, the small molecule compound was phosphorothioate oligonucleotide, a molar ratio of the phosphorothioate oligonucleotide to the initially modified primers was 0.6:1, and contents of the initially modified primers and the probes in the buffer solution were 10 µM.

The PCR mixed solution was purchased from NEB and added with Triton-X-100 with a final concentration of 0.1%, 1 U of thermally stable pyrophosphatase and 5 µg/µL of BSA, and further includes a PCR mix, where the PCR mix does not include a deoxycytidine triphosphate component.

The kit further includes positive quality control products and negative quality control products.

A preparation method for the positive quality control products includes: selecting 200 bp of a wild-type sequence and a mutant sequence at each gene mutation locus shown in Table 9, and loading the sequences into a plasmid vector pET-23d(+)(Promega) after synthesis, respectively; performing quantification by using Qubit 3.0, calculating a copy number concentration of two types of plasmids, and mixing the two plasmids at a copy number ratio of 1:3,000, 1:2,000, 1:1,000, 1:500, 1:200, 1:100, 1:50 and 1:10; and then, breaking a plasmid mixture into fragments of about 180 bp by ultrasonic treatment, and subjecting the fragments to quantification to 20 ng/µL to serve as the gradient positive quality control products. The negative quality control products are composed of the wild-type plasmid separately. Fragments of about 180 bp were obtained by breaking using a same method and then subjected to quantification to 20 ng/µL to serve as the negative quality control products.

The specific examples are only used for explaining this application and are not intended to limit this application. For those skilled in the art, modifications of the examples can be made without creative contribution according to needs after reading the description, and the modifications made within the scope of the claims of this application are protected under the patent law.

## Claims

1. A digital amplification detection method for simultaneously identifying multiple genotypes, **characterized by** comprising the following steps:
S1, extracting sample DNA;
and preparing a primer-probe mixed solution, comprising the following steps:
S11, synthesizing multiple primers and probes for amplification and detection of target genes, wherein for each of the target genes, at least two detection probes are designed and used for covering different regions of the target gene, and a nucleotide sequence for synthesizing the primers comprises four or less bases;
S12, adding a nucleotide sequence capable of forming a secondary structure at a 5' end of the primers, and modifying the primers with a small molecule compound to obtain primers for PCR amplification,
wherein the small molecule compound is selected from one or more of phosphorothioate oligonucleotide, methylated oligonucleotide, peptide nucleic acid, locked nucleic acid, hypoxanthine nucleotide or a rhodium metal inserting reagent;
S13, co-dissolving the primers for PCR amplification and the probes in a buffer solution for nucleic acid dissolution to obtain the primer-probe mixed solution;
S2, preparing a PCR reaction system and then carrying out a PCR reaction, wherein the PCR reaction system comprises the sample DNA and the primer-probe mixed solution; and carrying out the PCR reaction; and
S3, after the PCR reaction is completed, analyzing detection data to obtain genotypes at different loci.

2. The detection method according to claim 1, **characterized in that** the nucleotide sequence for synthesizing the primers comprises three bases and does not comprise a base C and a base G simultaneously.

3. The detection method according to claim 1, **characterized in that** the small molecule compound is connected to the primers by an additional addition method; and S12 comprises the following steps: adding a nucleotide sequence capable of forming a three-dimensional structure at the 5' end of the primers to obtain initially modified primers; adding the initially modified primers to a buffer solution, wherein the buffer solution comprises a small molecule compound; and carrying out a reaction to obtain the primers for PCR amplification.

4. The detection method according to claim 3, **characterized in that** a concentration of the small molecule compound in the buffer solution is 4-12 µM, and a molar ratio of the small molecule compound to the initially modified primers is (0.7-1):1.

5. The detection method according to claim 1, **characterized in that** a nucleotide sequence length of the probes is 10-30 bp.

6. The detection method according to claim 1, **characterized in that** the sample DNA comprises cfDNA.

7. The detection method according to claim 1, **characterized in that** in the primer-probe mixed solution, a concentration of each of the primers for PCR amplification is 7-12 µM, and a concentration of each of the probes is 7-12 µM.

8. The detection method according to claim 1, **characterized in that** when a reaction is carried out in step S12, heating is performed to 50-80°C first, and heat preservation is performed; and then cooling is performed to 28-32°C, and heat preservation is performed.

9. A detection product for detecting multiple genotypes by using the detection method according to any one of claims 1-8.

10. A detection kit for detecting multiple genotypes by using the detection method according to any one of claims 1-8.
